**Europäisches Patentamt**

**European Patent Office**

**Office européen des brevets**

(11) Veröffentlichungsnummer: **0 214 537 B1**

(12) **EUROPÄISCHE PATENTSCHRIFT**

(45) Veröffentlichungstag der Patentschrift:
31.10.90

(51) Int. Cl.⁵: **A61N 1/32**

(21) Anmeldenummer: 86111655.6

(22) Anmeldetag: 22.08.86

(54) **Reizstromgerät zur Erzeugung von mindestens zwei Wechselspannungen.**

(30) Priorität: 05.09.85 DE 3531739

(43) Veröffentlichungstag der Anmeldung:
18.03.87 Patentblatt 87/12

(45) Bekanntmachung des Hinweises auf die Patenterteilung:
31.10.90 Patentblatt 90/44

(84) Benannte Vertragsstaaten:
AT CH DE GB LI NL

(56) Entgegenhaltungen:
EP-A- 0 024 316
EP-A- 0 137 936
DE-A- 2 937 984
DE-A- 3 049 530

(73) Patentinhaber: Siemens Aktiengesellschaft,
Wittelsbacherplatz 2, D-8000 München 2(DE)

(72) Erfinder: Herzog, Ludwig, Drei-Thorn-Strasse 3,
D-6948 Waldmicheibach(DE)
Erfinder: Knapp, Volker, Pestalozzistrasse 19,
D-6948 Waldmicheibach(DE)

ACTORUM AG

**Beschreibung**

Die Erfindung betrifft ein Reizstromgerät zur Erzeugung von mindestens zwei Wechselspannungen gemäß Oberbegriff von Anspruch 1.

Ein derartiges Gerät ist aus der DE-A 30 49 530 bekannt. Die für jeden Behandlungsfall benötigten Amplitudenwerte werden hierbei jeweils von einem Mikrocomputer aufgrund von abgespeicherten Daten und Algorithmen berechnet und unter fortlaufenden Adressen in dem Stromformspeicher abgelegt. Daher läßt sich die Form der Wechselspannungen des Gerätes sowie die erzeugbaren Schwebungsfunktionen und Schwebungsfrequenzen bei relativ geringem Speicheraufwand in einem großen Bereich variieren. Dieses Konzept ist jedoch zu aufwendig, wenn lediglich die Schwebungsfrequenz der aus zwei Wechselspannungen resultierenden Schwebungsspannung in einem weiten Bereich veränderbar sein soll. Der Erfindung liegt daher die Aufgabe zugrunde, ein Reizstromgerät gemäß Oberbegriff von Anspruch 1 so weiterzubilden, daß es mit einem Stromformspeicher mit praktisch und wirtschaftlich angemessener Kapazität auskommt, und das trotzdem eine Variation der resultierenden Schwebungsfrequenz in einem weiten Bereich zuläßt.

Die erfindungsgemäße Lösung dieser Aufgabe ist in Anspruch 1 gekennzeichnet. Sie beruht auf einer phasenmodulierten Wechselspannung, die in Form von rechteckigen Perioden mit unterschiedlicher Phasenlage in einem vorprogrammierten Stromformspeicher relativ geringer Speicherkapazität abgespeichert ist.

Die Schwebungsfunktion, also der zeitliche Verlauf der Schwebungsspannung, hängt von der Lage der 1/0- und 0/1- Übergänge in den einzelnen aufeinanderfolgenden Speicherbereichen ab. Sie ist ferner durch die Zuordnung der Bereichsadressen zu den Speicherbereichen und durch die Auslesesteuerung, also die Folge der Bereichsadressen und/oder der Platzadressen und den Auslesetakt, variierbar: So kann jeder Speicherbereich mehrfach ausgelesen oder ein Speicherbereich übersprungen werden. Auch ist ein Sprung von einer Platzadresse eines Speicherbereiches auf die folgende Platzadresse eines anderen Speicherbereiches möglich. Durch die Kombination solcher Maßnahmen läßt sich die Schwebungsfunktion in weitem Bereich variieren, wobei die Schwebungsfrequenz zusätzlich von der gewählten Taktfrequenz abhängig ist.

Auch der Phasenhub ist - bei entsprechender Auslegung des Stromformspeichers in einfacher Weise steuerbar. Um z.B. einen Phasenhub von 2 zu erhalten, braucht man nur diejenigen Speicherbereiche des Stromformspeichers zyklisch auszulesen, die in einem Winkelbereich ε symmetrisch zu einem mittleren Speicherbereich mit der Phasenlage 0 liegen.

Es ist ein Wesensmerkmal der Erfindung, daß bei ihr - im Gegensatz zum vorgenannten Stand der Technik - dem Stromformspeicher kein Digitalanaloglogwandler nachgeschaltet ist, da man beim Auslesen des Stromformspeichers bereits eine phasenmodulierte Rechteckspannung erhält. Diese braucht lediglich noch verstärkt zu werden, wobei gegebenenfalls dem Verstärker Filter zur Umformung in eine Sinusspannung vorgeschaltet sein können.

Für die Erfindung ist in erster Linie ein Stromformspeicher (ROM, RAM, EPROM) geeignet, der je Adresse nur eine Speicherzelle -also nur ein Bit - hat. Gemäß einer Weiterbildung der Erfindung sind aber auch übliche Speicher mit a Speicherzellen je Adresse - also Speicher mit a Bit je Speicherplatz - brauchbar. Die a Ausgangsleitungen eines solchen Speichers sind dann gemäß Anspruch 2 über einen a stelligen Stellenschalter mit der Ausgangsklemme des jeweiligen Kanals verbunden, wobei der Stellenschalter einen codierten Steuereingang hat, der an einen Teil der niederwertigsten Stellen des Bereichsadreßgebers angeschlossen ist. In diesem Fall bilden also die a Speicherzellen jedes Speicherplatzes (jeder Sprachadresse) eine Gruppe von a Speicherbereichen, denen eine gemeinsame Gruppenadresse zugeordnet ist. Nach jedem Durchlauf der Platzadressen wird zunächst nur der Stellenschalter von einer Ausgangsleitung auf die nächste weitergeschaltet und so der Inhalt der a Speicherbereiche nacheinander ausgelesen. Erst nach dem Umschalten des Stellenschalters von der letzten Ausgangsleitung auf die erste, wird eine neue Gruppenadresse ausgegeben und die nächste Bereichsgruppe von a Speicherbereichen ausgelesen.

Der Bereichsadreßgeber, der die Bereichsadressen für den Stromformspeicher und gegebenenfalls die Schaltbefehle für einen dem Stromformspeicher nachgeschalteten Stellenschalter liefert, enthält vorzugsweise einen Binärzähler, dessen Stellenzahl z gleich ld n ist, wobei n die Zahl der Speicherbereiche (= Zahl der abzuspeichernden Perioden) und ld der Logarithmus zur Basis 2 bedeuten. Ein solcher Binärzähler ist hier als Bereichsadreßzähler bezeichnet. Sein Eingang ist an eine Steuereinrichtung angeschlossen, die Zählimpulse zur Fortschaltung des Bereichsadreßzählers liefert. Diese Steuereinrichtung ist vorzugsweise ein peripherer Baustein eines Mikrocomputers, z.B. in Form eines programmierbaren Zählers.

Der Platzadreßgeber ist vorzugsweise ebenfalls ein Binärzähler mit r=ld m Stellen, der zyklisch von 0 bis m durchzählt, wobei m die Zahl der Speicherplätze je Speicherbereich ist. In einem solchen Fall ist es besonders zweckmäßig, eine der Wechselspannungen direkt von der höchstwertigen Stelle dieses Zählers abzugreifen.

Ein Ausführungsbeispiel der Erfindung wird anhand der Figuren näher erläutert; es zeigen

FIG 1 des Prinzipschaltbild eines Reizstromgerätes mit drei Kanälen,

FIG 2 ein erstes Beispiel für die Belegung der Speicherplätze eines Stromformspeichers und

FIG 3 ein zweites Beispiel einer Speicherbelegung.

Zwei Kanäle mit den Ausgangsklemmen A2 und A3 des Reizstromgerätes sind identisch ausgeführt: Jeder Kanal hat einen Stromformspeicher SF2 (SF3) - z.B. ein EPROM - mit a Bit je Adresse, mit einem Adreßeingang SF2E (SF3E) und mit einem Ausgang SF2A (SF3A) mit a Stellen. An letzteren ist über eine a-polige Ausgangsleitung AL2 (AL3) ein Stellenschalter SS2 (SS3) angeschlossen, der einen binären Steuereingang mit x=ld a Steuerleitungen hat. Der Ausgang des Stellenschalters ist über eine Verstärkereinrichtung V an die Ausgangsklemme A2 (A3) angeschlossen. Die Verstärkereinrichtung enthält einen Schaltverstärker VS und ein oder zwei nachgeschaltete Aktivfilter VF, wie dies anhand des untersten Kanals gezeigt ist. Die Schaltverstärker VS aller Verstärkereinrichtungen V sind über Entkopplungswiderstände R an den Ausgang eines Steuerverstärkers RS angeschlossen, der die Betriebsspannung für die Schaltverstärker vorgibt: Dadurch ist die Größe des rechteckigen Ausgangssignales in allen drei Kanälen gleichzeitig steuerbar.

In jedem der Stromformspeicher sind n volle Perioden - mit unterschiedlicher Phasenlage - abgelegt, wobei je Periode m Speicherplätze vorgesehen sind. Zur Abfrage der m Speicherplätze sind im Binärsystem r=ld m Stellen erforderlich. Dementsprechend ist ein Platzadreßgeber PAG in Form eines Binärzählers mit r Ausgängen über einen Platzadreßbus PAB mit den r niederwertigen Stellen der Adreßeingänge beider Stromformspeicher SF2, SF3 verbunden.

Zur Abfrage der Speicherbereiche jedes Stromformspeichers dient ein Bereichsadreßzähler BAZ2 (BAZ3), der einen Adreßausgang mit z=ld n Stellen hat. Von diesen Stellen sind x=ld a niederwertige Stellen über Steuerleitungen SL2 (SL3) mit dem Steuereingang des zugehörigen Stellenschalters SS2 (SS3) verbunden. Die höherwertigen y Stellen des Bereichsadreßzählers sind über einen Bereichsadreßbus BAB2 (BAB3) mit den höherwertigen Stellen des Adreßeinganges des Stromformspeichers SF2 (SF3) verbunden; dabei ist y = z - x = ld n - ld a = ld M, wobei M = n/a die Zahl der Gruppenadressen bedeutet.

Die Zählimpulse zum Weiterschalten erhält jeder Bereichsadreßzähler BAZ2 (BAZ3) von einem zugeordneten Bereichssteuerteil BST2 (BST3), das jeweils an den Datenbus DB, den Adreßbus AB und einen Steuerbus SB eines Mikrocomputers angeschlossen ist. Jeder Bereichssteuerteil besteht im wesentlichen aus einem programmierbaren Zähler.

Der Mikrocomputer umfaßt in bekannter Weise einen Mikroprozessor µP, einen Arbeitsspeicher AS, einen Festwertspeicher FS, ein Eingabefeld EF sowie ein Anzeigefeld AF. Ferner sind eine Uhr - nicht dargestellt - eine Überwachungseinheit UE sowie ein Taktgeber T vorgesehen. Als spezifische Peripherie sind die bereits erwähnten Bereichssteuerteile BST2 (BST3) sowie ein Digitalanalogwandler DAW mit einem zugeordneten Adreßdekodierer AD vorgesehen: Der Digitalanalogwandler liefert an den Steuerverstärker RS den Sollwert für die Größe der Wechselspannungen an den Ausgangsklemmen A1 bis A3 entsprechend der jeweiligen Eingabe über das Eingabefeld EF.

Der Taktgeber T liefert einen Grundtakt von 2,56 MHz an den Mikroprozessor µP und Zählimpulse mit einer Folgefrequenz von 1,28 MHz an den Platzadreßgeber PAG: Dieser gibt also in diesem Takt zyklisch die Platzadressen für die m Speicherplätze der beiden Stromformspeicher SF2, SF3 aus.

Jeder der Bereichssteuerteile BST2 (BST3) erhält Zündimpulse von einem eigenen Taktgeber oder von dem Taktgeber T und liefert Schaltimpulse an den jeweils nachgeschalteten Bereichsadreßzähler BAZ2 (BAZ3). Der Abstand aufeinanderfolgender Schaltimpulse ist dabei durch den Mikrocomputer bestimmt: Dieser gibt über seinen Datenbus DB Zahlenwerte aus, durch die die zeitliche Folge der Schaltimpulse bestimmt ist. Diese Zahlenwerte hängen ihrerseits ab von der jeweiligen Eingabe über das Eingabefeld EF sowie von im Festwertspeicher FA abgelegten Daten und Programmen; damit ist auch eine zeitliche Änderung der Schwebungsfrequenz möglich.

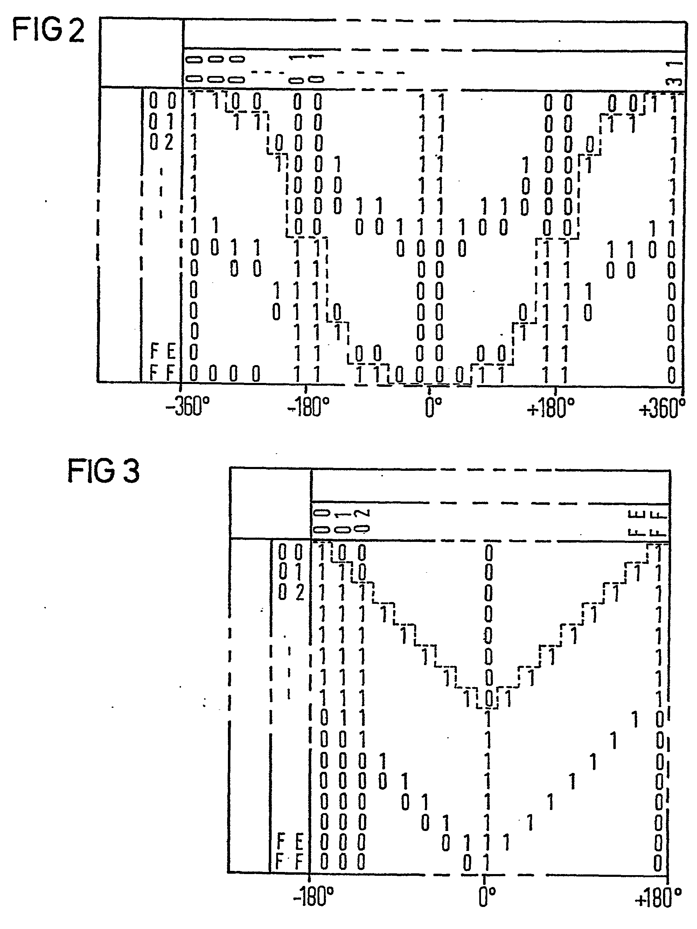

Die FIG 2 und 3 veranschaulichen die Speicherorganisation der Stromformspeicher SF2 (SF3) in Form einer Matrix: Jeder Spalte der Matrix ist eine Bereichsadresse und jeder Zeile eine Platzadresse zugeordnet. Jede Spalte enthält eine volle Periode der Wechselspannung als Folge von 1 und 0. Diese Folge wird bei der zyklischen Ausgabe der Platzadressen periodisch ausgelesen, wodurch eine Rechteckspannung entsteht, deren 0-Durchgänge durch die Lage der Übergänge von 1 auf 0 und 0 auf 1 in jeder Spalte bestimmt sind. Werden nun die Spalten nacheinander ausgelesen und liegen die Übergänge 0/1 und 1/0 auf verschiedenen Platzadressen, so resultiert daraus eine Rechteckschwingung, deren Phasenlage sich ändert. Wird z.B. die in FIG. 2 dargestellte Speicherbelegung zyklisch Spalte für Spalte von links nach rechts ausgelesen, so ändert sich die Phasenlage von -360° bis +360° nach einer etwa sinusförmigen Funktion (vgl. die gestrichelte Treppenkurve). Nach derselben Funktion verläuft dann eine Schwebungsspannung, die aus der Überlagerung der so gewonnenen phasenmodulierten Spannung mit einer unmodulierten Wechselspannung gleicher Mittenfrequenz - an Ausgangsklemme A1 - resultiert. Letztere Wechselspannung wird direkt von der höchstwertigen Stelle der r-stelligen Adreßausganges des Platzadreßgebers PAG abgegriffen.

Die in FIG 2 dargestellte Form der Phasenmodulation erfordert mitunter dieselbe Lage der 1/0- und 0/1-Übergänge in mehreren Spalten. Dasselbe Ergebnis kann aber - bei kleinerem Speicherbedarf - auch durch eine entsprechende Steuerung des Bereichssteuerteils BST vom Mikrocomputer aus dadurch erreicht werden, daß dieselbe Spalte mehrmals nacheinander ausgelesen wird, bevor der Bereichsadreßzähler BAZ auf die nächste Spalte weiterschaltet. Mit der gleichen Maßnahme ist bei gleichbleibendem Auslesetakt auch eine Halbierung der Schwebungsfrequenz erreichbar. Eine Verdoppelung der Schwebungsfrequenz wird nach demselben Prinzip dadurch herbeigeführt, daß nur die Hälfte der Platzadressen der einen Spalte und dann die andere Hälfte von Platzadressen aus einer anderen Spalte ausgelesen werden.

Wird der Bereichsadreßzähler BAZ2 (BAZ3) von dem zugeordneten Bereichssteuerteil BST2 (BST3) und dem Mikrocomputer so gesteuert, daß alle Spalten nacheinander zyklisch ausgelesen werden, dann ergibt sich bei der in FIG 2 dargestellten Speicherorganisation ein Phasenhub von ± 360°. Es ist aber auch jeder kleinere Phasenhub dadurch realisierbar, daß - veranlaßt durch die Steuerung - nur Spalten zyklisch ausgelesen werden, die symmetrisch zu der Mitte des Speichers liegen; die Mitte ist in FIG 2 durch den Hinweis 0° markiert. Für einen Phasenhub von ± 180° wären dann dementsprechend die Spalten von den Markierungen -180° bis +180° zyklisch auszulesen.

Bei der Speicherbelegung nach FIG 2 wurde davon ausgegangen, daß der Stromformspeicher SF2 (SF3) je Speicherplatz a (acht) Speicherzellen hat; dementsprechend bilden a Spalten eine Bereichsgruppe mit derselben Gruppenadresse. Die a Spalten (Speicherbereiche) mit derselben Gruppenadresse werden mit Hilfe des Stellenschalters SS2 (SS3) der Reihe nach ausgelesen, bevor auf die nächst höhere Gruppenadresse umgeschaltet wird. Hierzu liegen die drei niederwertigsten Ausgänge des Bereichsadreßzählers BAZ2 (BAZ3) an dem jeweiligen Stellenschalter SS2 (SS3), der dadurch gesteuert der Reihe nach jeden der a Ausgänge des Stromformspeichers an die Verstärkereinrichtung V schaltet. Da die höherwertigen Stellen des Bereichsadreßzählers an den höherwertigen Stellen des Adreßeinganges des Stromformspeichers liegen, wird durch diese immer erst dann auf die nächst höhere Gruppenadresse des Stromformspeichers umgeschaltet, wenn der Stellenschalter nach Durchlauf über alle a Ausgangsleitungen wieder in die Anfangsstellung zurückkehrt. Dementsprechend werden zur Abspeicherung von n Perioden nur M = n/a Gruppenadressen benötigt. Bei n=256 und a=8 resultieren hieraus 32 Gruppenadressen (0 bis 31).

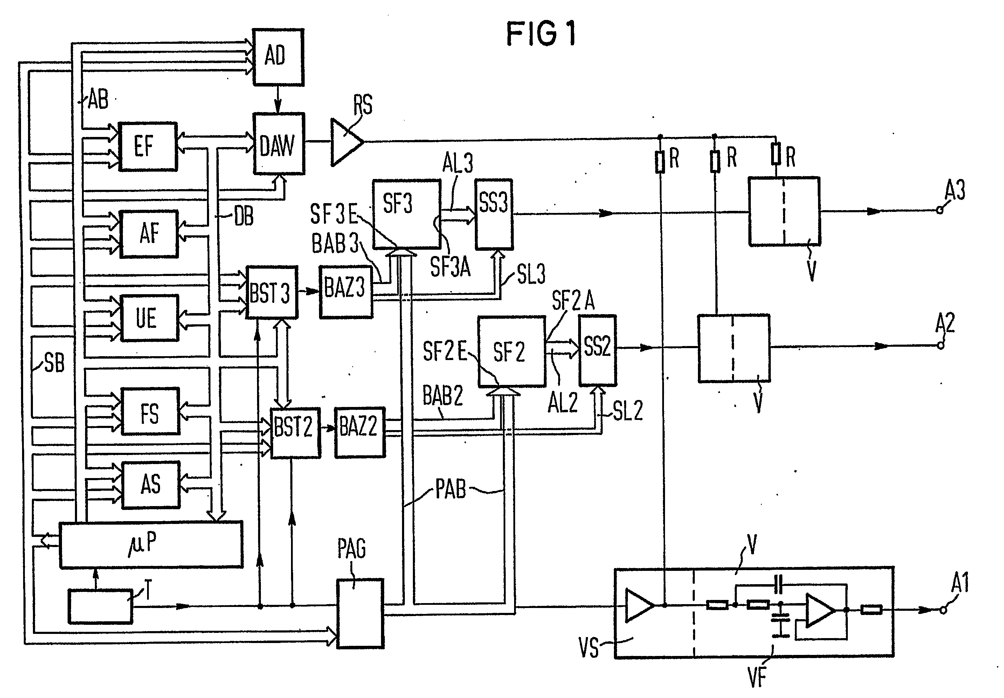

Wird abweichend von dieser Speicherbelegung und der in FIG 1 dargestellten Ausführungsform ein Stromformspeicher verwendet, der je Platzadresse nur eine Speicherzelle (a=1) hat, dann muß nach Auslesen aller Platzadressen (00 bis FF) eines Bereiches der Bereichsadreßzähler auf die nächste Bereichsadresse umschalten. Hierzu müssen alle z Stellen des Bereichsadreßgebers auf den Adreßeingang des Stromformspeichers geschaltet sein, der hierzu ebenfalls z Stellen haben muß. Sollen auch hier 256 Perioden abgespeichert werden, so sind gleich viele Speicherbereiche mit ihren Adressen 00 bis FF vorzusehen, wie dies FIG 3 zeigt. Diese Speicherorganisation veranschaulicht zugleich einen Fall mit einem Phasenhub von nur ± 180°. Die Schwebungsfunktion hat hierbei einen dreieckigen Verlauf - siehe gestrichelte Treppenkurve.

Begriffsliste

| | |
|---|---|
| AB | Adreßbus |
| | Adreßausgang |
| | Adreßeingang |
| | Adreßgeber |
| AD | Adreßdekodierer |
| AF | Anzeigefeld |
| AS | Arbeitsspeicher |
| A1, A2, A3 | Ausgangsklemmen |
| AL2, AL3 | Ausgangsleitung |
| | Bereichsadresse |
| BAB2, BAB3 | Bereichsadreßbus |
| | Bereichsadreßgeber |
| BAZ2, BAZ3 | Bereichsadreßzähler |
| | Bereichsgruppe |
| BST2, BST3 | Bereichssteuerteil |
| DAW | Digitalanalogwandler |
| DB | Datenbus |
| EF | Eingabefeld |
| FS | Festwertspeicher |
| | Gruppenadresse |
| | Kanal |
| | Mikrocomputer |
| µP | Mikroprozessor |
| | Patientenstromkreis |
| | Platzadresse |
| PAB | Platzadreßbus |
| PAG | Platzadreßgeber |
| RS | Steuerverstärker |
| | Reizstromgerät |
| SB | Steuerbus |
| | Schwebungsspannung |
| | Schwebungsfrequenz |
| | Schwebungsfunktion |
| SF2, SF3 | Stromformspeicher |
| | Speicherbereich |
| | Speicherplatz |
| | Speicherzelle |
| | Steuereingang |
| | Steuereinrichtung |
| SL2, SL3 | Steuerleitungen |
| SS2, SS3 | Stellenschalter |
| T T | aktgeber |
| UE | Überwachungseinheit |
| V | Verstärkereinrichtung |
| VF | Aktivfilter |
| VS | Schaltverstärker |
| n | Zahl der Perioden (Speicherbereiche) |
| m | Zahl der Speicherplätze je Speicherbereich |
| a | Zahl der Speicherzellen je Speicherplatz (Adresse) |

## Patentansprüche

1. Reizstromgerät zur Erzeugung von mindestens zwei Wechselspannungen, die überlagert eine Schwebungsspannung mit einer Schwebungsfunktion und einer Schwebungsfrequenz ergeben,
- mit mindestens zwei Kanälen zur Erzeugung dieser Wechselspannungen an Ausgangsklemmen (A1, A2, A3) für daran anschließbare Partientenstromkreise,
- mit einem Stromformspeicher (SF2, SF3) in mindestens einem der Kanäle, in dem der Verlauf der Wechselspannung als Folge von Amplitudenwerten gespeichert ist und der einen Adreßeingang aufweist,
- mit einem Adreßgeber, dessen Adreßausgang an den Adreßeingang des Stromformspeichers angeschlossen ist und der die Amplitudenwerte aus dem Stromformspeicher (SF2, SF3) zyklisch abruft, und
- mit einem Taktgeber (T),
**dadurch gekennzeichnet,**
- daß der Stromformspeicher (SF2, SF3) n Speicherbereiche mit zugeordneten Bereichsadressen und jeder Speicherbereich m Speicherplätze mit zugeordneten Platzadressen hat,
- daß in den m Speicherplätzen jedes Speicherbereiches die Amplitudenwerte einer vollen Periode der Wechselspannung als Folge von 1 und 0 gespeichert sind, wobei die Schwebungsfunktion durch die Lage der beiden Übergänge 0/1 bzw. 1/0 in den Speicherplätzen der n Speicherbereiche bestimmt ist,
- daß der Adreßgeber einen Platzadreßgeber (PAG) und einen Bereichsadreßgeber (BST + BAZ) aufweist,
- daß der Adreßausgang des Platzadreßgebers (PAG) über einen Platzadreßbus (PAB) an die niederwertigen Stellen des Adreßeinganges des Stromformspeichers (SF2, SF3) angeschlossen ist,
- daß der Platzadreßgeber (PAG) eingangsseitig an den Taktgeber (T) angeschlossen ist und durch diesen gesteuert die Adressen für die m Speicherplätze zyklisch ausgibt, und
- daß der Adreßausgang des Bereichsadreßgebers über einen Bereichsadreßbus (BAB2, BAB3) an die höherwertigen Stellen des Adreßeinganges des Stromformspeichers angeschlossen ist.

2. Reizstromgerät nach Anspruch 1, **dadurch gekennzeichnet,**
- daß der Stromformspeicher (SF2, SF3) je Adresse a Speicherzellen hat, von denen jede an eine von a Ausgangsleitungen (AL2, AL3) angeschlossen ist,
- daß zwischen den a Ausgangsleitungen des Stromformspeichers und der Ausgangsklemme (A1, A2, A3) eines Kanals ein Stellenschalter (SS2, SS3) mit Steuereingängen angeordnet ist,
- daß ein Teil der niederwertigen Stellen des Adreßausganges des Bereichsadreßgebers über Steuerleitungen (SL2, SL3) an die Steuereingänge des Stellenschalters (SS2, SS3) angeschlossen sind,
- und daß nur die verbleibenden höherwertigen Stellen des Adreßausganges des Bereichsadreßgebers an die höherwertigen Stellen des Adreßeinganges des Stromformspeichers angeschlossen sind.

3. Reizstromgerät mit einer Verstärkereinrichtung (V) in jedem Kanal, die der Ausgangsklemme (A1 bis A3) vorgeschaltet ist, nach Anspruch 1 oder 2, **dadurch gekennzeichnet,** daß die Verstärkereinrichtung (V) eines der Kanäle eingangsseitig an die höchstwertige Stelle des Adreßausganges des Platzadreßgebers (PAG) angeschlossen ist.

4. Reizstromgerät nach Anspruch 3, **dadurch gekennzeichnet,** daß ein gemeinsamer Platzadreßgeber (PAG) für die Stromformspeicher (SF2, SF3) von mindestens zwei Kanälen vorgesehen ist.

5. Reizstromgerät nach einem der Ansprüche 1 bis 4, **dadurch gekennzeichnet,** daß der Bereichsadreßgeber einen Bereichsadreßzähler (BAZ2, BAZ3) mit dem Bereichsadreßausgang sowie eine Steuereinrichtung für den Bereichsadreßzähler enthält.

6. Reizstromgerät nach Anspruch 5, **dadurch gekennzeichnet,** daß die Steuereinrichtung für den Bereichsadreßzähler (BAZ2, BAZ3) einen Mikrocomputer mit einem Mikroprozessor (μP), einem Arbeitsspeicher (AS), einem Festwertspeicher (FS), einem Anzeigefeld (AF), einem Eingabefeld (EF) und einem Bereichssteuerteil (BST2, BST3) enthält, und daß der Bereichssteuerteil ausgangsseitig an einen Steuereingang des Bereichsadreßzählers (BAZ2, BAZ3) angeschlossen ist.

7. Reizstromgerät nach Anspruch 6, **dadurch gekennzeichnet,** daß die Verstärkereinrichtung (V) jedes Kanales einen Schaltverstärker (VS) enthält, der seine Betriebsspannung über einen Entkopplungswiderstand (R) von einem gemeinsamen Steuerverstärker (RS) erhält, und daß der Steuerverstärker eingangsseitig an den Ausgang eines Digital-Analog-Wandlers (DAW) angeschlossen ist, der eingangsseitig an den Datenbus (DB) und über einen Adreßdekoder (AD) an den Adreßbus (AB) und den Steuerbus (SB) des Mikrocomputers angeschlossen ist.

**Claims**

1. An exciter current apparatus for generating at least two alternating voltages, which yield, superimposed, a beating voltage with a beating function and a beat frequency,
- with at least two channels for generating these alternating voltages at output terminals (Al, A2, A3) for the part current circuits able to be connected thereto,
- with a current waveform store (SF2, SF3) in at least one of the channels, in which the shape of the alternating voltage is stored as a series of amplitude values and which has an address input,
- with an address generator, the address output of which is connected to the address input of the current waveform store and which retrieves cyclically the amplitude values from the current waveform store (SF2, SF3) and
- with a clock generator (T), characterised
- in that the current waveform store (SF2, SF3) has n storage areas with allocated area addresses and each storage area has m storage locations with allocated location addresses,
- in that in the m storage locations of each storage area the amplitude values of a full period of the alternating voltage are stored as a series of 1s and 0s, wherein the beating function is determined by the positions of the two transitions 0/1 or 1/0 in the storage locations of the n storage areas,
- in that the address generator has a location address generator (PAG) and an area address generator (BST + BAZ),
- in that the address output of the location address generator (PAG) is connected by way of a location address bus (PAB) to the low-value locations of the address input of the current waveform store (SF2, SF3),
- in that the location address generator (PAG) is connected on the input side to the clock generator (T), and, controlled by this, outputs in cycles the addresses for the m storage locations, and
- in that the address output of the area address generator is connected by way of an area address bus (BAB2, BAB3) to the higher-value locations of the address input of the current waveform store.

2. An exciter current apparatus according to claim 1, characterised

- in that the current waveform store (SF2, SF3) has per address a storage locations, each of which is connected to one of a output lines (AL2, AL3),

- in that arranged between a output lines of the current waveform store and the output terminal (A1, A2, A3) of a channel there is a positional switch (SS2, SS3) with control inputs,

- in that a part of the low-value locations of the address output of the area address generator is connected by way of control lines (SL2, SL3) to the control inputs of the positional switch (SS2, SS3),

- in that only the remaining higher-value locations of the address output of the area address generator are connected to the higher-value locations of the address input of the current waveform store.

3. An exciter current apparatus with an amplifier device (V) in each channel, which is upstream of the output terminal (A1 to A3), according to claim 1 or 2, characterised in that the amplifier device (V) of one of the channels is connected on the input side to the highest-value location of the address output of the location address generator (PAG).

4. An exciter current apparatus according to claim 3, characterised in that a common location address generator (PAG) is provided for the current waveform store (SF2, SF3) of at least two channels.

5. An exciter current apparatus according to claims 1 to 4, characterised in that the area address generator contains an area address counter (BAZ2, BAZ3) with the area address output as well as a control device for the area address counter.

6. An exciter current apparatus according to claim 5, characterised in that the control device for the area address counter (BAZ2, BAZ3) contains a microcomputer with a microprocessor (MP), a working store (AS), a fixed value store (FS), an indicator field (AF), an input field (EF) and an area control part (BST2, BST3), and in that the area control part is connected on the output side to a control input of the area address counter (BAZ2, BAZ3).

7. An exciter current apparatus according to claim 6, characterised in that the amplifier device (V) of each channel contains a switch amplifier (VS), which receives its operational voltage by way of a decoupling resistance (R) from one common control amplifier (RS) and in that the control amplifier is connected on the input side to the output of a digital-analog-converter (DAW), which is connected on the input side to the data bus (DB) and by way of an address decoder (AD) to the address bus (AB) and the control bus (SB) of the microcomputer.

## Revendications

1. Appareil à courant d'excitation pour produire au moins deux tensions alternatives qui fournissent, en superposition, une tension de battement avec une fonction de battement et avec une fréquence de battement,

– avec au moins deux canaux pour produire ces tensions de battement aux bornes de sortie (A1, A2, A3) pour des circuits de courants de patients susceptibles d'y être raccordés,

– avec une mémoire de conformation du courant (SF2, SF3) dans au moins l'un des canaux, dans laquelle est mémorisée l'allure de la tension alternative en fonction de valeurs des amplitudes, et qui comporte une entrée d'adresse,

– avec un générateur d'adresses, dont la sortie d'adresse est reliée à l'entrée d'adresse de la mémoire de conformation du courant, et qui appelle cycliquement les valeurs de l'amplitude de la mémoire de conformation du courant, et

– avec un générateur de cadence (T), caractérisé par le fait,

– que la mémoire de conformation du courant (SF2, SF3) possède n zones de mémoire avec des adresses de zones associées et que chaque zone de mémoire possède m emplacements de mémoire avec des adresses d'emplacement associées,

– que dans les m emplacements de mémoire de chaque zone de mémoire, sont mémorisées les valeurs des amplitudes d'une période complète de la tension alternative sous la forme d'une série de 1 et de 0, la fonction de battement étant déterminée par la position des deux transitions 0/1 et 1/0 dans les emplacements de mémoire des n zones de mémoire,

– que le générateur d'adresses comporte un générateur d'adresses d'emplacements (PAG) et un générateur d'adresses de zones (BST + BAZ),

– que la sortie d'adresse du générateur d'adresses d'emplacement (PAG) et relié, par l'intermédiaire d'un bus d'adresses d'emplacements (PAB), aux emplacements, de poids le plus faible, de l'entrée d'adresse de la mémoire de conformation du courant (SF2, SF3),

– que le générateur d'adresses d'emplacements (PAG) est relié, côté entrée, au générateur de cadence (T), et il émet cycliquement, en étant commandé par ce dernier, les adresses pour les n emplacements de mémoire, et

– que la sortie d'adresse du générateur d'adresses de zones est relié, par l'intermédiaire d'un bus d'adresses de zones (BAB2, BAB3), aux emplacements, de poids le plus élevé, de l'entrée d'adresse de la mémoire de conformation du courant.

2. Appareil à courant d'excitation selon la revendication 1, caractérisé par le fait,

– que la mémoire de conformation du courant (SF2, SF3) comporte, par adresse, a cellules de mémoire dont chacune est réliée à l'un de a conducteurs de sortie (AL2, AL3),

– qu'entre les a conducteurs de sortie de la mémoire de conformation du courant et la borne de sortie (A1, A2, A3) d'un canal, est disposé un interrupteur de positions (SS2, SS3) avec des entrées de commande,

– qu'une partie des positions de poids le plus faible de l'entrée d'adresse du générateur d'adresses de zones, est reliée, par l'intermédiaire de conducteurs de commande (SS2, SS3), aux entrées de commande de l'interrupteur de positions (SS2, SS3),
– et que seulement les positions de poids le plus élevé qui subsistent, de la sortie d'adresse du générateur d'adresses de zones, sont reliées aux positions ou emplacements de poids le plus élevé de l'entrée d'adresse de la mémoire de conformation du courant.

3. Appareil à courant d'excitation avec un dispositif amplificateur (V) dans chaque canal, monté en amont de la borne de sortie (A1 à A3), selon la revendication 1 ou 2, caractérisé par le fait que le dispositif amplificateur (V) de l'un des canaux est relié, côté entrée, à la position de poids le plus élevé de la sortie d'adresse du générateur d'adresses d'emplacements (PAG).

4. Appareil à courant d'excitation selon la revendication 3, caractérisé par le fait qu'il est prévu un générateur d'adresses d'emplacements commun (PAG) pour les mémoires de conformation du courant (SF2, SF3) d'au moins deux canaux.

5. Appareil à courant d'excitation selon l'une des revendications 1 à 4, caractérisé par le fait que le générateur d'adresses de zones comporte un compteur d'adresse de zones (BAZ2, BAZ3), avec la sortie d'adresse de zones, ainsi qu'un dispositif de commande pour le compteur d'adresses de zones.

6. Appareil à courant d'excitation selon la revendication 5, caractérisé par le fait que le dispositif de commande pour le compteur d'adresses de zones (BAZ2, BAZ3) comporte un micro-ordinateur avec un micro-processeur (MP), une mémoire de travail (AS), une mémoire morte (FS), une plage d'indications (AF), une plage d'introductions (EF) et un élément de commande de zones (BST2, BST3), et que l'élément de commande de zones est relié, côté sortie, à une entrée de commande du compteur d'adresse de zones (BAZ2, BAZ3).

7. Appareil à courant d'excitation selon la revendication 6, caractérisé par le fait que le dispositif amplificateur (V) de chaque canal comporte un amplificateur de commutation (VS) qui reçoit sa tension de fonctionnement, par l'intermédiaire d'une résistance de découplage (R), à partir d'un amplificateur de commande commun, et que l'amplificateur de commande est relié, côté entrée, à la sortie d'un convertisseur numérique-analogique (DAW) qui est relié, côté entrée, au bus de données (DB) et, par l'intermédiaire d'un décodeur d'adresse (AD), au bus d'adresses (AB) et au bus de commande (SB) du micro-ordinateur.

FIG 1

FIG 2

−360°    −180°    0°    +180°    +360°

FIG 3

−180°    0°    +180°